# EUROPÄISCHE PATENTSCHRIFT

(11) **EP 0 980 885 B1**
(45) Veröffentlichungstag und Bekanntmachung des Hinweises auf die Patenterteilung: **24.08.2005**
(21) Anmeldenummer: 99111997.5
(22) Anmeldetag: 29.06.1999
(51) Int. Cl.: C08G 77/04, C08L 83/04, A61K 7/48, A61K 7/06

(54) **Mischungen flüchtiger linearer Siloxane**
Mixtures of volatile, linear siloxanes
Mélanges de composés de siloxanes volatiles et linéaires

(30) Priorität: 20.08.1998 DE 19837850
(43) Veröffentlichungstag der Anmeldung: 23.02.2000
(73) Patentinhaber: Wacker-Chemie GmbH, D-81737 München (DE)
(72) Erfinder: Koini, Thomas, Dr., 84489 Burghausen (DE); Köhler, Thomas, Dr., 84556 Kastl (DE); Huber, Annemarie, 84553 Haiming (DE); Meisenberger, Manfred, 84489 Burghausen (DE); Berthiaume, Marianne D., Tecumseh, Michigan 49286 (US)
(74) Vertreter: Fritz, Helmut

(56) Entgegenhaltungen:
- EP-A- 0 400 546
- EP-A- 0 515 082
- EP-A- 0 774 482
- WO-A-98/32418
- DE-A- 3 319 764
- FR-A- 2 771 003
- US-A- 4 331 167

## Beschreibung

Die Erfindung betrifft Mischungen linearer Organosiloxane, Verfahren zu ihrer Herstellung sowie ein Verfahren zur Herstellung von kosmetischen Formulierungen.

Flüchtige Siloxanverbindungen finden ein breites Anwendungsfeld in einer Vielzahl von Bereichen, u.a. in der Kosmetik als flüchtige Trägersubstanz. Aufgrund ihrer geringen Verdampfungswärme verdunsten flüchtige Siloxanverbindungen in der Regel, ohne ein Kältegefühl oder ein Brennen auf der Haut zu bewirken.

Die flüchtigen Siloxanverbindungen verdunsten aufgrund des hohen Dampfdrucks und des niedrigen Siedepunkts schnell und rückstandslos. Die Verdampfungsrate darf aber nicht so groß sein, daß keine Zeit zum gleichmäßigen Auftragen, Verteilen, Verreiben oder Einarbeiten der die flüchtigen Siloxanverbindungen enthaltenden Formulierung auf Haut, Haar, etc. bleibt. Außerdem sollen die behandelten Stellen dabei nicht ausgetrocknet werden, sondern glatt und geschmeidig werden.

Am häufigsten werden ausschließlich Methylgruppen enthaltende cyclische Siloxane mit 4 bis 6 Siloxangruppen (Kurzbezeichnungen D₄, D₅, D₆), insbesondere deren Gemische eingesetzt, da diese eine günstige Verdampfungsrate aufweisen. Jedoch steht D₄ im dringenden Verdacht, eine reproduktionstoxische Wirkung aufzuweisen.

In US-A-5,002,762 und US-A-5,084,577 sind vielfältig substituierte lineare Siloxane für den Einsatz in der Kosmetik beschrieben. Siloxanmischungen mit einem günstigen Abdampfverhalten sind nicht offenbart. Die linearen Siloxane von US-A-5,002,762 enthalten als Reste funktionelle Gruppen, welche in kosmetischen Formulierungen unerwünschte und unüberschaubare Wechselwirkungen eingehen können.

In WO 98/32418 A, DE 3319764 A, US 4331167 A, EP 400546 A und EP 774482 A sind einzelne Organosiloxane und Mischungen von flüchtigen linearen Organosiloxanen für die Verwendung in kosmetischen Zusammensetzungen beschrieben. In EP 515082 A ist die Herstellung von Mischungen linearer Organosiloxane beschrieben.

Der Erfindung liegt die Aufgabe zugrunde, flüchtige Trägersubstanzen für die Kosmetik bereitzustellen, welche keine cyclischen Siloxane mit 4 bis 6 Siloxangruppen aufweisen, möglichst reaktionsträge sind und eine gute Kompatibilität mit einer Vielzahl anderer kosmetischer Rohstoffe besitzen.

Gegenstand der Erfindung sind Mischungen linearer Organosiloxane der allgemeinen Formel 1

R₃SiO-(SiR₂O)ₙ-SiR₃ (1),

in der
- **R**: Methylrest bedeutet und
- **n**: Werte von 0 bis 20 aufweist , wobei n bei
6 bis 12 Gew.-% der Organosiloxane den Wert 0, bei
45 bis 55 Gew.-% der Organosiloxane den Wert 1, bei
22 bis 30 Gew.-% der Organosiloxane den Wert 2, bei
6 bis 12 Gew.-% der Organosiloxane den Wert 3, bei
1 bis 5 Gew.-% der Organosiloxane den Wert 4, bei
0 bis 3 Gew.-% der Organosiloxane den Wert 5, und bei
0 bis 1 Gew.-% der Organosiloxane die Werte 6 bis 20
aufweist, und
wobei die Mischungen gemessen nach DIN 53249 nach
30 Minuten zu 80 bis 95 Gew.-%, nach
60 Minuten zu 90 bis 99 Gew.-% und nach
120 Minuten zu 95 bis 100 Gew.-% verdampft sind.

Die Erfindung beruht auf der Entdeckung, daß die Mischungen linearer Organosiloxane sogar ein erheblich günstigeres Abdampfverhalten als die cyclischen Siloxane mit 4 bis 6 Siloxangruppen aufweisen.

Die Verdampfungsraten nach DIN 53249 werden gemessen, indem
1. ein rundes Filterpapier mit Durchmesser 150 mm gewogen wird,
2. mit einer Pipette 0,3 ml Probe aufgebracht wird, das Filter sofort gewogen wird und
3. in 5 min-Abständen bei RT (25°C) an einem zugfreien Ort das Filter erneut gewogen wird.

Es wird jeweils auf 0,001 g genau gewogen.

**n** weist vorzugsweise Werte von höchstens 12, insbesondere höchstens 10 auf.

Vorzugsweise sind die Mischungen gemessen nach DIN 53249 nach 5 Minuten zu 20 bis 80 Gew.-%, insbesondere zu 30 bis 70 Gew.-% und nach 15 Minuten zu 60 bis 90 Gew.-% insbesondere zu 70 bis 85 Gew.-% verdampft.

Vorzugsweise sind die Mischungen gemessen nach DIN 53249 nach 30 Minuten zu 85 bis 94 Gew.-%, nach 60 Minuten zu 94 bis 97 Gew.-% und nach 120 Minuten zu 97 bis 99,5 Gew.-% verdampft.

Die Mischungen linearer Organosiloxane werden vorzugsweise dadurch hergestellt, daß ein Gemisch aus 1 Teil Trimethylchlorsilan und 2,5 bis 6 Teilen, vorzugsweise 3, 5 bis 4,5 Teilen Dimethyldichlorsilan in Salzsäure hydrolysiert wird.

Vorzugsweise werden in einem zweiten Schritt überschüssige Organosiloxane der allgemeinen Formel 1, in der **n** die Werte 0 bis 2 hat, abgetrennt, vorzugsweise durch Destillation. Bei der Hydrolyse wird vorzugsweise die Konzentration der Salzsäure durch Zudosieren von Wasser konstant gehalten. Vorzugsweise ist die Konzentration der Salzsäure 15 bis 25 Gew.-%.

Die Mischungen linearer Organosiloxane werden bei der Herstellung von kosmetischen Formulierungen zugesetzt. Die Mischungen linearer Organosiloxane finden vorzugsweise Verwendung in einer Vielfalt von kosmetischen Anwendungen, beispielsweise in Formulierungen aus dem Bereich der Haarpflege, wie Haarspray, Shampoo, Mousse, Styling Gel, Styling Lotion, Conditioner, Haarfarben, Haarbleichmittel, etc.; Formulierungen aus dem Bereich Antiperspirantien und Deodorantien; Formulierungen aus dem Bereich der Hautpflege, wie Körperlotionen, Handcremes, Feuchtigkeitscremes, Babycremes, etc; Formulierungen aus dem Bereich des Sonnenschutzes, wie Sonnencremes, Sonnenmilch, Lippenschutz, etc. und Formulierungen aus dem Bereich der Deckenden Kosmetik, wie Lippenstift, Mascara, Gesichtspuder, Foundation, etc.

Die Mischungen linearer Organosiloxane unterstützen bei der gleichmäßigen Verteilung aktiver Einsatzstoffe auf Haut und Haar. In der dekorativen Kosmetik bieten sie trotz rascher Verdunstung ausreichend Zeit zur Verarbeitung, und verbessern hierdurch die Auftrageigenschaften, vermindern die Klebrigkeit und machen die Haut glatter und geschmeidiger. In Haarpflege-Applikationen unterstützen flüchtige Siloxanverbindungen die gleichmäßige Verteilung von hochviskosen Oelen, verbessern die Naßkämmbarkeit und wirken als Harzplastifizierer in Haarfestigern/Styling-Produkten.

Bei einem Großteil der kosmetischen Formulierungen werden Duftstoffe eingesetzt. Die Mischungen linearer Organosiloxane bewirken durch ihr schnelles Abdampfverhalten zu Beginn; insbesondere bis 30 min gemäß DIN 53249, daß die Kopf-Note des verwendeten Duftstoffes besonders schnell und ausgeprägt zur Geltung kommt. Durch die reduzierte Abdampfgeschwindigkeit nach 30 min gemäß DIN 53249 und die besonders stark reduzierte Abdampfgeschwindigkeit nach 60 min gemäß DIN 53249 bleibt die Basisnote des Duftstoffes besonders lange erhalten.

Anhand der folgenden Beispiele wird die Erfindung näher erläutert. Alle Angaben von Teilen und Prozentsätzen beziehen sich auf das Gewicht. Die Beispiele werden bei einem Druck der umgebenden Atmosphäre, also etwa bei 0,1 MPa, und bei Raumtemperatur, also etwa bei 21 °C, durchgeführt.

### Beispiele

### Beispiel 1 (Herstellung) (nicht erfindungsgemäß)

In einem Rundkolben werden 100 g einer 20 Gew.-%igen HCl-Lösung vorgelegt.
Unter Rühren werden innerhalb von 60 Minuten 560 g eines molaren 4 : 1- Gemisches von Dimethyldichlorsilan und Trimethylchlorsilan dosiert.
Parallel wird Wasser dosiert, so daß die Konzentration der wäßrigen Salzsäure konstant bleibt.
Anschließend läßt man 60 Minuten nachrühren.
Nach Phasentrennung wird die organische Phase mit Wasser neutral gewaschen.
Das erhaltene Rohprodukt wird durch Destillation von der Hauptmenge des bei der Hydrolyse/Kondensation gebildeten Hexamethyldisiloxans befreit.
Man erhält ein Endprodukt mit der folgenden Zusammensetzung, wobei **n** der allgemeinen Formel angegeben ist:
20 Gew.-% weisen den Wert 0,
43 Gew.-% weisen den Wert 1,
24 Gew.-% weisen den Wert 2,
8 Gew.-% weisen den Wert 3,
3 Gew.-% weisen den Wert 4,
1 Gew.-% weisen den Wert 5, und
1 Gew.-% der Organosiloxane weisen die Werte 6 bis 10 auf.

### Beispiel 2 (Verdunstungsraten)

In nachstehender Tabelle sind die Verdunstungsraten aufgeführt, welche nach DIN 53249 ermittelt wurden.

| | D₅ | 80 Gew.% D₄ 20 Gew.% D₅ | D₄ | Wasserdest. | Beispiel 1 |
|---|---|---|---|---|---|
| Zeit (min) | | | | | |
| | | | | | |
| 5 | 3,51 | 19,58 | 28,64 | 27,12 | 47,49 |
| 10 | 8,05 | 36,25 | 53,15 | 48,29 | 70,43 |
| 15 | 13,34 | 53,13 | 72,11 | 72,43 | 78,49 |
| 20 | 17,76 | 69,41 | 92,85 | 89,02 | 84,85 |
| 25 | 23,45 | 79,23 | 99,41 | 97,26 | 87,88 |
| 30 | 28,49 | 86,02 | 100,00 | 98,25 | 90,67 |
| 45 | 45,64 | 96,18 | | 98,06 | 93,33 |
| 60 | 61,98 | 100,00 | | 98,59 | 96,05 |
| 90 | 89,77 | | | 98,58 | 97,38 |
| 120 | 99,01 | | | 97,52 | 98,46 |
| 180 | | | | | 99,91 |

Im Vergleich zu den Vorteilen der Mischung von Beispiel 1 die Eigenschaften der Cyclen:
D₄ ist gemäß DIN 53249 nach 30 min verdampft und auch die weitverbreiteten D₄/D₅-Gemische (ca. 80/20) nach ca. 60 min. Ebenso ist das Abdampfverhalten durch eine geringere Abdampfgeschwindigkeit bestimmt als bei der Mischung von Beispiel 1 . Zum Vergleich: D₅ ist nach 120 min zu 99% abgedampft; nach 30 min jedoch erst zu ca. 29% und nach 60 min zu ca. 62%, wodurch sich bei der Anwendung der Formulierung ein nasses, öliges Gefühl nicht vermeiden läßt.

### Beispiel 3 (Kosmetische Formulierungen mit der Mischung von Beispiel 1)

In der Verarbeitung war kein entscheidender Unterschied im Vergleich zu Cyclen (D₄, D₅ und 80/20-D₄/D₅-Gemisch) auszumachen. Das optische Erscheinungsbild der Formulierungen blieb unverändert.

### a) Antiperspirant Stift

Fester Stift mit wenig weichem Abrieb.
A 18,00 % Stearinsäure
   18,00 % Cetylalkohol
   24,00 % Aluminiumchlorhydrat
B 40,00 % Mischung von Beispiel 1
   nach Bedarf Duftstoffe, Farbstoffe

A vermischen und auf 75 - 80°C erhitzen, B in A einmischen.

Vergleich: Mischung von Beispiel 1 wird ersetzt durch gleiche Menge an D₅.
Anmerkung: Bei Formulierung a) trockeneres Hautgefühl nach kürzerer Zeit.

### b) Deo Pumpspray

Farblos, klar, dünnflüssig
30,00 % Ethanol
69,00 % Mischung von Beispiel 1
1,00 % Duftstoff

Alle Komponenten gut vermischen.

Vergleich: Mischung von Beispiel 1 wird ersetzt durch gleiche Menge an D₅.
Anmerkung: Formulierung b) zieht schneller ein als Vergleich; angenehm trockenes Hautgefühl nach kürzerer Zeit.

### c) Badeöl

Farblos, leicht trüb, dünnflüssig.
25,00 % Mischung wie Beispiel 1
70,00 % Avocadoöl
5,00 % PPG-15 Stearylether
nach Bedarf Konservierungsmittel,
Farbstoffe, Duftstoffe

Alle Komponenten mischen.

Vergleich: Mischung von Beispiel 1 wird ersetzt durch gleiche Menge an 80/20-D₄/D₅-Gemisch.
Anmerkung: Formulierung c) zeigt bessere Kompatibilität als Vergleich; zieht besser ein.

### d) Sonnenschutzöl

Farblos, klar, dünnflüssig.
- A: 10,00 % Mischung wie Beispiel 1
10,00 % Isopropylmyristat
77,00 % Paraffinöl
- B: 3,00 % Octyl Methoxycinnamate
nach Bedarf Konservierungsmittel,
Duftstoffe, Farbstoffe

A mischen, B zugeben, mischen.

Vergleich: Mischung von Beispiel 1 wird ersetzt durch gleiche Menge an 80/20-D₄/D₅-Gemisch.
Anmerkung: Formulierung d) zieht schneller ein als Vergleich; früher angenehm trockenes Hautgefühl

### e) Sonnenschutzöl

Farblos, klar, dünnflüssig.
- A: 40,00 % Mischung wie Beispiel 1
10,00 % Isopropylmyristat
47,00 % Oleyl Oleate
- B: 3,00 % Octyl Methoxycinnamate
nach Bedarf Konservierungsmittel,
Duftstoffe, Farbstoffe

A mischen, B zugeben, mischen.

Vergleich: Mischung von Beispiel 1 wird ersetzt durch gleiche Menge an 30 % D₅ und 10 % Hexamethyldisiloxan.
Anmerkung: Formulierung e) zieht schneller ein als Vergleich; früher angenehm trockenes Hautgefühl.

### f) Gesichtspuder

Homogener Puder.
- A: 74,00 % Talkum
6,00 % Magnesiumstearat
3,00 % Acrylate / C₁₀₋₃₀ Alkyl Acrylat Crosspolymer
- B: 12,00 % Mischung wie Beispiel 1
2,00 % Hydrolized Animal Protein (INCI)
- C: 0,20 % Methylparaben
1,90 % Talkum
0,70 % Pigmente
nach Bedarf Duftstoffe, Farbstoffe

A gut vermischen, B portionsweise einmischen, C zugeben, D homogen einmischen.

Vergleich: Mischung von Beispiel 1 wird ersetzt durch gleiche Menge an D₅.
Anmerkung: Formulierung f) ist feiner verteilbar auf der Haut als Vergleich; keine Klumpen-/Schichtenbildung

### g) Pflegecreme

Weiße feste Creme, gut zu verteilen, samtiges Hautgefühl.
- A: 3,85 % Polysorbate-60
7,69 % Cetearyl Alcohol
19,23 % Petrolatum (INCI)
- B: 38,46 % Wasser
7,69 % Glycerin
- C: 23,08 % Mischung wie Beispiel 1
nach Bedarf Konservierungsmittel,
Duftstoffe

A und B jeweils auf 70°C erhitzen. B in A einmischen.

### h) Deostift

Cremefarbener Stift mit weichem Abrieb.
- 1,00 %: Zinc Ricinoleate,
Triethanolamine, Dipropylene
Glycol, Lactic Acid
- 8,50 %: Natriumstearat
- 5,50 %: Glycerin
- 4,00 %: Mischung wie Beispiel 1
- 70,40 %: Propylenglykol
- 10,00 %: Ethanol
- 0,60 %: Hydroxyethylcellulose
nach Bedarf Duftstoffe, Farbstoffe

Alle Komponenten vermischen und aufschmelzen. Die homogene Lösung heiß abfüllen.

Vergleich: Mischung von Beispiel 1 wird ersetzt durch gleiche Menge an D₅.
Anmerkung: Bei Formulierung h) wird ein trockeneres Hautgefühl früher erreicht.

### i) Cover cream

Weiche homogene Creme.
- A: 5,50 % Candelillawachs
6,70 % Stearoxy Dimethicone (INCI)
3,00 % Stearinsäure
- B: 44,80 % Wasser
3,40 % Propylenglykol
1,30 % Triethanolamin
- C: 14,00 % Titandioxid
- D: 18,30 % Mischung von Beispiel 1
nach Bedarf Konservierungsmittel,
Parfüm, Farbstoffe

A und B je auf 70°C erhitzen. B zu A mischen. C homogen einarbeiten. Etwas abkühlen lassen, bei ca 30°C D einrühren.

Vergleich: Mischung von Beispiel 1 wird ersetzt durch gleiche Menge an D₅.

Anmerkung: Formulierung i) ist besser verteilbar und wasserfester als Vergleich.

### k) Deodorant Stift

Fester, leicht gelblicher Stift mit weichem Abrieb.
- 50,00 %: Wollwachssäure
- 36,00 %: Stearoxy Dimethicon (INCI)
- 5,00 %: Isopropylmyristat
- 4,00 %: Dimethicon, Viskosität 350 mm²/sec
- 5,00 %: Mischung wie Beispiel 1
nach Bedarf Duftstoffe, Farbstoffe

Alle Komponenten gemeinsam aufschmelzen.

Vergleich: Mischung von Beispiel 1 wird ersetzt durch gleiche Menge an D₅.

Anmerkung: Formulierung f) zeigt etwas weniger und weicheren Abrieb als Vergleichsprobe.

### 1) Badeöl

Gelblich, klar, dünnflüssig.
- A: 1,00 % Stearoxy Dimethicon (INCI)
69,00 % Jojobaöl
25,00 % Mischung wie Beispiel 1
5,00 % Polypropylenglykol-15 Stearylether
nach Bedarf Konservierungsmittel,
Farbstoffe, Duftstoffe

A auf 50°C erhitzen, B in A einmischen.

Vergleich: Mischung von Beispiel 1 wird ersetzt durch gleiche Menge an D₅.

Anmerkung: Formulierung 1) zeigt bessere Verteilung im Badewasser.

### m) Sonnenschutzöl

Gelblich, klar, dünnflüssig.
- 60,00 %: Mischung wie Beispiel 1
- 10,00 %: Dimethiconol, Dimethicon Viskosität > 10 mm²/s
- 10,00 %: Diisopropyladipat
- 7,00 %: Octyl Methoxycinnamat
- 3,00 %: Benzophenon-3
- 10,00 %: C₁₂₋₁₅ Alkyl Benzoat
nach Bedarf Konservierungsmittel,
Duftstoffe, Farbstoffe

Alle Komponenten gut vermischen und filtrieren.

Vergleich: Mischung von Beispiel 1 wird ersetzt durch gleiche Menge an D₅.

Anmerkung: Formulierung m) zieht schneller ein als Vergleich, angenehm trockenes Hautgefühl nach kürzerer Zeit.

## Patentansprüche

1. Mischungen linearer Organosiloxane der allgemeinen Formel 1
R₃SiO-(SiR₂O)ₙ-SiR₃ (1),
in der
**R** Methylrest bedeutet und
**n** Werte von 0 bis 20 aufweist , wobei n bei
6 bis 12 Gew.-% der Organosiloxane den Wert 0, bei
45 bis 55 Gew.-% der Organosiloxane den Wert 1, bei
22 bis 30 Gew.-% der Organosiloxane den Wert 2, bei
6 bis 12 Gew.-% der Organosiloxane den Wert 3, bei
1 bis 5 Gew.-% der Organosiloxane den Wert 4, bei
0 bis 3 Gew.-% der Organosiloxane den Wert 5, und bei
0 bis 1 Gew.-% der Organosiloxane die Werte 6 bis 20
aufweist, und
wobei die Mischungen gemessen nach DIN 53249 nach
30 Minuten zu 80 bis 95 Gew.-%, nach
60 Minuten zu 90 bis 99 Gew.-% und nach
120 Minuten zu 95 bis 100 Gew.-% verdampft sind.

2. Mischungen nach Anspruch 1 , bei denen n Werte von höchstens 12 aufweist.

3. Mischungen nach Anspruch 1 oder 2, bei denen die Mischungen, gemessen nach DIN 53249 nach 5 Minuten zu 20 bis 80 Gew.-%, und nach 15 Minuten zu 60 bis 90 Gew.-% verdampft sind.

4. Verfahren zur Herstellung der Mischungen gemäß Anspruch 1 bis 3, bei dem ein Gemisch aus 1 Teil Trimethylchlorsilan und 2,5 bis 6 Teilen Dimethyldichlorsilan in Salzsäure hydrolysiert wird.

5. Kosmetische Formulierungen, die Mischungen gemäß Anspruch 1 bis 3 enthalten.

## Claims

1. Mixtures of linear organosiloxanes of the general formula 1
R₃SiO-(SiR₂O)ₙ-SiR₃ (1),
where
R is a methyl radical, and
n has values from 0 to 20, where n in
6 to 12% by weight of the organosiloxanes has the value 0, in
45 to 55% by weight of the organosiloxanes has the value 1, in
22 to 30% by weight of the organosiloxanes has the value 2, in
6 to 12% by weight of the organosiloxanes has the value 3, in
1 to 5% by weight of the organosiloxanes has the value 4, in
0 to 3% by weight of the organosiloxanes has the value 5, and in
0 to 1% by weight of the organosiloxanes has the value 6 to 20, and
where the mixtures have evaporated, measured in accordance with DIN 53249,
to an extent of from 80 to 95% by weight after 30 minutes,
to an extent of from 90 to 99% by weight after 60 minutes and
to an extent of from 95 to 100% by weight after 120 minutes.

2. Mixtures according to Claim 1, in which n has values of at most 12.

3. Mixtures according to Claim 1 or 2, in which the mixtures have evaporated, measured in accordance with DIN 53249, to an extent of from 20 to 80% by weight after 5 minutes, and to an extent of from 60 to 90% by weight after 15 minutes.

4. Process for the preparation of the mixtures of Claim 1 to 3, in which a mixture of 1 part of trimethylchlorosilane and from 2.5 to 6 parts of dimethyldichlorosilane is hydrolysed in hydrochloric acid.

5. Cosmetic formulations which comprise mixtures of Claims 1 to 3.

## Revendications

1. Mélanges d'organosiloxanes linéaires de formule générale 1
R₃SiO-(SiR₂O)ₙ-SiR₃ (1)
dans laquelle
**R** représente le radical méthyle et
**n** a des valeurs de 0 à 20, **n** ayant dans
6 à 12 % en poids des organosiloxanes la valeur 0, dans
45 à 55 % en poids des organosiloxanes la valeur 1, dans
22 à 30 % en poids des organosiloxanes la valeur 2, dans
6 à 12 % en poids des organosiloxanes la valeur 3, dans
1 à 5 % en poids des organosiloxanes la valeur 4, dans
0 à 3 % en poids des organosiloxanes la valeur 5, et dans
0 à 1 % en poids des organosiloxanes les valeurs 6 à 20,
et,
mesuré selon DIN 53249, les mélanges étant évaporés après
30 minutes à raison de 80 à 95 % en poids, après
60 minutes à raison de 90 à 99 % en poids et après
120 minutes à raison de 95 à 100 % en poids.

2. Mélanges selon la revendication 1, dans lesquels n a des valeurs de 12 au maximum.

3. Mélanges selon la revendication 1 ou 2, dans lesquels, mesuré selon DIN 53249, les mélanges sont évaporés après 5 minutes à raison de 20 à 80 % en poids, et après 15 minutes à raison de 60 à 90 % en poids.

4. Procédé pour la préparation des mélanges selon l'une quelconque des revendications 1 à 3, dans lesquels un mélange de 1 partie de triméthyl-chlorosilane et de 2,5 à 6 parties de diméthyldichlorosilane est hydrolysé dans de l'acide chlorhydrique.

5. Compositions cosmétiques qui contiennent des mélanges selon l'une quelconque des revendications 1 à 3.
